Europäisches Patentamt

European Patent Office

Office européen des brevets

(11) Numéro de publication: **0 562 021 B1**

# FASCICULE DE BREVET EUROPEEN

(45) Date de publication du fascicule du brevet: **08.11.95**  (51) Int. Cl.6: **A61B 5/046**, A61N 1/39

(21) Numéro de dépôt: **92903226.6**

(22) Date de dépôt: **13.12.91**

(86) Numéro de dépôt internationale :
**PCT/FR91/01011**

(87) Numéro de publication internationale :
**WO 92/10805 (25.06.92 92/14)**

(54) **PROCEDE DE RECONNAISSANCE D'UN ETAT PATHOLOGIOUE CARDIAOUE VENTRICULAIRE EN VUE D'UNE DEFIBRILLATION AUTOMATIOUE.**

(30) Priorité: **13.12.90 FR 9015804**

(43) Date de publication de la demande:
**29.09.93 Bulletin 93/39**

(45) Mention de la délivrance du brevet:
**08.11.95 Bulletin 95/45**

(84) Etats contractants désignés:
**AT BE CH DE DK ES FR GB IT LI LU NL SE**

(56) Documents cités:
**FR-A- 2 103 913**
**US-A- 4 453 551**
**US-A- 4 523 595**

**PROCEEDINGS OF THE ANNUAL INT. CONF. OF THE IEEE ENG. IN MEDICINE AND BIOLO-GY SOCIETY vol. 10, no. 3, 7 Novembre 1988, NEW ORLEANS, US pages 1303 - 1304; K. KRANTZ ET AL.: 'MEDICALE USAGE OF AN EXPERT SYSTEM FOR RECOGNIZING CHAOS'**

(73) Titulaire: **ODAM, S.A.**
**34 Rue de l'Industrie**
**F-67160 Wissembourg (FR)**

(72) Inventeur: **FELBLINGER, Jacques**
**Rehhag 17**
**CH-3018 Berne (CH)**
Inventeur: **CANSELL, Albert**
**7, rue des Bergers**
**F-67160 Wissembourg (FR)**
Inventeur: **MEYER, Didier**
**32, rue du Roi-de-Rome**
**F-67160 Wissembourg (FR)**

(74) Mandataire: **Nuss, Pierre et al**
**10, rue Jacques Kablé**
**F-67080 Strasbourg Cédex (FR)**

PROCEEDINGS OF COMPUTERS IN CARDIO-LOGY 28 Septembre 1988, WASHINGTON, US pages 325 - 328; ZHU YI-SHENG ET AL.: 'DE-TECTION OF VENTRICULAR FIBRILLATION BY SEOUENTIAL TESTING'

IEEE TRANSACTIONS ON BIOMEDICAL ENGI-NEERING vol. BME25, no. 4, Juillet 1978, NEW YORK, US pages 353 - 361; D.E.GUSTAFSON ET AL.: 'ECG/VCG RHYTHM DIAGNOSIS USING STATISTICAL SIGNAL ANALYSIS-II. IDENTIFICATION OF TRANSIENT RHYTHMS'

**Description**

La présente invention se rapporte à un procédé de surveillance, de détection et de reconnaissance automatique d'un état pathologique cardiaque dangereux sur un patient tel qu'une fibrillation ventriculaire ou une tachycardie ventriculaire en vue d'une alerte puis d'une défibrillation semi-automatique.

Elle peut en particulier être mise en oeuvre dans un moniteur-détecteur-défibrillateur.

La surveillance de l'activité cardiaque s'avère nécessaire dans tous les cas pathologiques et cardiaques graves et dans tous les cas d'urgence dans la mesure où elle permet de renseigner sur l'état momentané du patient.

De plus, les patients et malades sujets à des troubles cardiaques, et notamment à des troubles du rythme cardiaque, nécessitent une surveillance attentive dépendant de la gravité de leur état.

En effet, l'arythmie conduit généralement à des troubles graves pouvant, dans certaines conditions, aboutir à la fibrillation du myocarde, état gravissime que seul un choc électrique de défibrillation peut modifier.

Une fibrillation correspond à une désynchronisation totale de l'excitation des fibres cardiaques, due à des boucles d'excitation qui se referment sur elles-mêmes. Un mouvement auto-entretenu est créé dans ces boucles, appelées cercles de reentrée, empêchant toute nouvelle excitation du muscle cardiaque.

Une fibrillation localisée dans les oreillettes (fibrillation auriculaire) n'est pas mortelle et peut être réduite par un choc électrique.

Localisée dans les ventricules, cette fibrillation (fibrillation ventriculaire) stoppe entièrement le fonctionnement du coeur. En effet, la contraction mécanique du coeur ne se fait pratiquement plus. Cette totale inefficacité hémodynamique provoque la mort dans les trois à cinq minutes après le début du trouble en raison d'un manque d'irrigation du cerveau. Seul un choc de défibrillation électrique peut resynchroniser l'ensemble des cellules du coeur.

Ce traitement consiste à appliquer à travers le thorax, par deux électrodes, un courant de faible durée de quelques dizaines d'ampères sous quelques milliers de volts durant quelques millisecondes résultant de la décharge d'un condensateur.

Depuis quelques années, les défibrillateurs sont associés à un moniteur cardiaque afin de visualiser le signal d'électrocardiogramme, en abrégé ECG, avant et après le choc de défibrillation.

Actuellement, la fibrillation ventriculaire est responsable de la plupart des décès survenant au cours de la phase pré-hospitalière d'infarctus du myocarde, faute d'intervention rapide et de matériel d'urgence approprié. En effet, les détecteurs d'arythmie actuels sont complexes, non transportables, et nécessitent la présence d'un médecin pour reconnaître la pathologie de fibrillation.

Le premier but de l'invention est donc de réaliser procédé pouvant être mis en oeuvre dans un matériel d'urgence, simple, autonome, léger, consommant peu d'énergie et qui soit capable de détecter et de reconnaître automatiquement un état de fibrillation ventriculaire en vue d'émettre une alarme rendant ainsi la présence d'un médecin non indispensable pour la reconnaissance de la fibrillation.

Un autre but est d'étendre la reconnaissance à d'autres pathologies à risques comme la tachycardie ventriculaire de fréquence cardiaque supérieure à 140 p/mn, qui présentent des critères de reconnaissance communs avec ceux de la fibrillation et nécessitent également un choc électrique pour son traitement.

Ainsi un des buts de l'invention consiste à proposer, pour un moniteur-détecteur, un procédé capable de reconnaître aussi bien la fibrillation ventriculaire que la tachycardie ventriculaire.

Un but annexe de l'invention est de pouvoir délivrer une information de détection du complexe QRS synchronisé correctement avec l'électrocardiogramme en raison de l'importance du moment du choc pour la tachycardie ventriculaire.

Un autre but de l'invention concerne la possibilité, dès que l'alarme est émise, de pouvoir préparer le choc électrique et de proposer une décision de choc, c'est à dire de placer le défibrillateur d'urgence associé en situation de délivrer le choc dès que l'opérateur valide la proposition, raccourcissant ainsi notablement le délai d'intervention.

Encore un autre but de l'invention concerne la possibilité d'une décision de défibrillation automatique après une analyse complémentaire.

Un but supplémentaire de l'invention est celui de pouvoir fonctionner en temps réel.

Le dernier but précisé ici consiste à concevoir un procédé de détection de la fibrillation utilisable dans un moniteur-détecteur dont le fonctionnement présente le moins de faux négatifs correspondant à des détections manquantes et le moins de faux positifs correspondant à des détections en trop.

Pour atteindre tous ces buts, il a fallu identifier et résoudre les nombreuses difficultés de la détection automatique de la fibrillation et de la tachycardie ventriculaires, y remédier et imaginer entièrement un procédé d'analyse et de reconnaissance de la pathologie à partir des signaux d'électrocardiogramme.

Il a fallu notamment surmonter les importantes difficultés suivantes :

. recherches et études du choix des critères d'analyse et de reconnaissance ;

. éliminer ou neutraliser tous les parasites faussant la détection et l'identification tels que signaux induits, interférants ou de couplage avec les appareils associés, bruits de fond, parasites apportés par le réseau, radiofréquences du milieu et des impulsions de stimulation ;

. créer un logiciel d'analyse et de reconnaissance ;

. établir, pour chaque critère, une échelle des probabilités croissantes.

On connaît déjà par le document US-A-4453551 un appareil d'analyse et de traitement du signal ECG après échantillonnage, mettant en oeuvre un procédé de surveillance, de détection et de reconnaissance d'un état cardiaque pathologique à risques, tel que décrit dans le préambule de la revendication 1.

Toutefois, les valeurs des critères d'analyse déterminées ne sont soumises qu'à un unique test à deux niveaux (tout ou rien), ne permettant pas de tenir compte de la valeur de l'écart entre la valeur mesurée d'un critère donné et la valeur seuil correspondante.

En outre, cet appareil connu ne permet pas de déterminer spécifiquement une tachycardie ventriculaire.

Or, la présente invention a pour objet un procédé de surveillance, de détection et de reconnaissance d'un état cardiaque pathologique à risques du type mentionné dans le préambule de la revendication 1, caractérisé en ce qu'il consiste à subdiviser chaque durée D de mesure et d'analyse répétitive en deux périodes élémentaires consécutives identiques TA et TB, à établir des valeurs de critères de zéro Z et de degré d'arythmie RR, ainsi qu'une valeur de critère de fréquence cardiaque FC, pour chaque période élémentaire TA et TB, à affecter à chacune de ces valeurs de critères Z, RR et FC une valeur de probabilité élémentaire propre, fonction des niveaux desdites valeurs et de leur situation sur une échelle de pondération à plusieurs niveaux propre à chaque critère et, à sommer les probabilités élémentaires précitées de chaque période élémentaire d'analyse immédiate TB avec celles de la période élémentaire d'analyse antérieure TA, pour obtenir, en temps réel et en permanence, une probabilité globale PGV pour chaque durée D de mesure et d'analyse composée de deux périodes élémentaires, permettant, d'une part, de reconnaître un état pathologique nécessitant une défibrillation, par comparaison de la valeur de PGV avec une valeur limite ou seuil de probabilité prédéterminée, et, d'autre part, le cas échéant, de déclencher une alarme d'urgence et de mettre un défibrillateur associé en condition de charge électrique en vue d'une validation de décharge de défibrillation, une valeur de probabilité supplémentaire PTV étant, en outre, additionnée à chaque probabilité élémentaire lorsque la valeur du critère RR est inférieure à une valeur prédéterminée et au moins un temps d'interbattement Ti sur n est supérieure à une durée fixée.

Les caractéristiques techniques et d'autres avantages de l'invention sont consignés dans la description qui suit, effectuée à titre d'exemple non limitatif sur un mode d'exécution en référence aux dessins accompagnants dans lesquels :

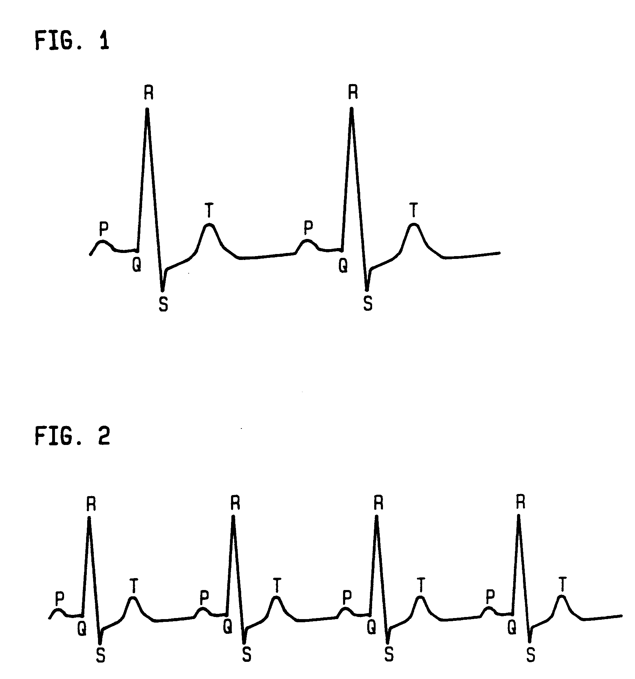

. la figure 1 est la forme d'onde agrandie d'un électrocardiogramme ;

. la figure 2 est un électrocardiogramme normal, c'est-à-dire la forme d'onde des signaux électrocardiographiques dans le cas d'une activité cardiaque normale ;

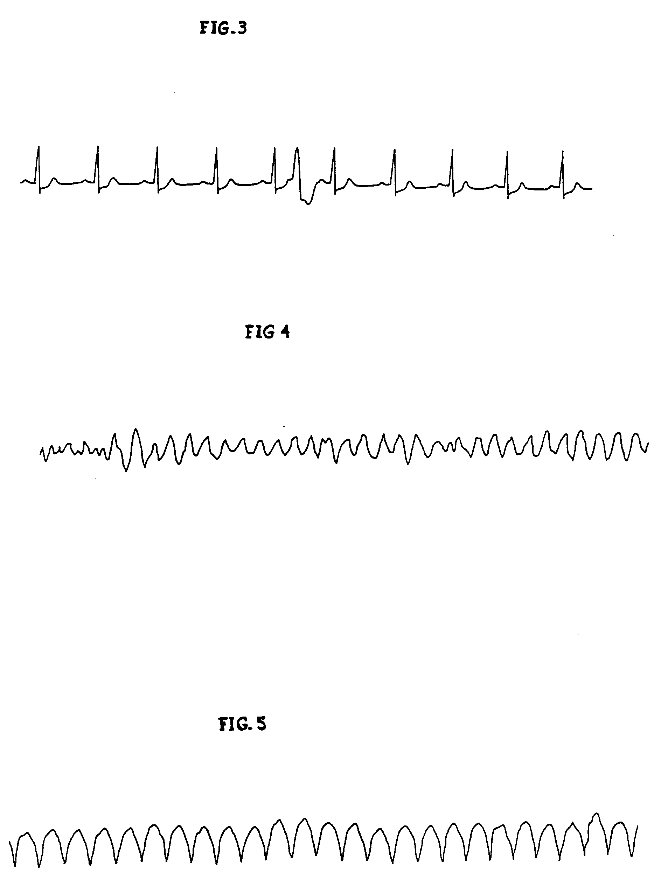

. la figure 3 est un électrocardiogramme normal montrant des formes d'onde correspondant à des extrasystoles ;

. la figure 4 est un électrocardiogramme d'un état de fibrillation ventriculaire ;

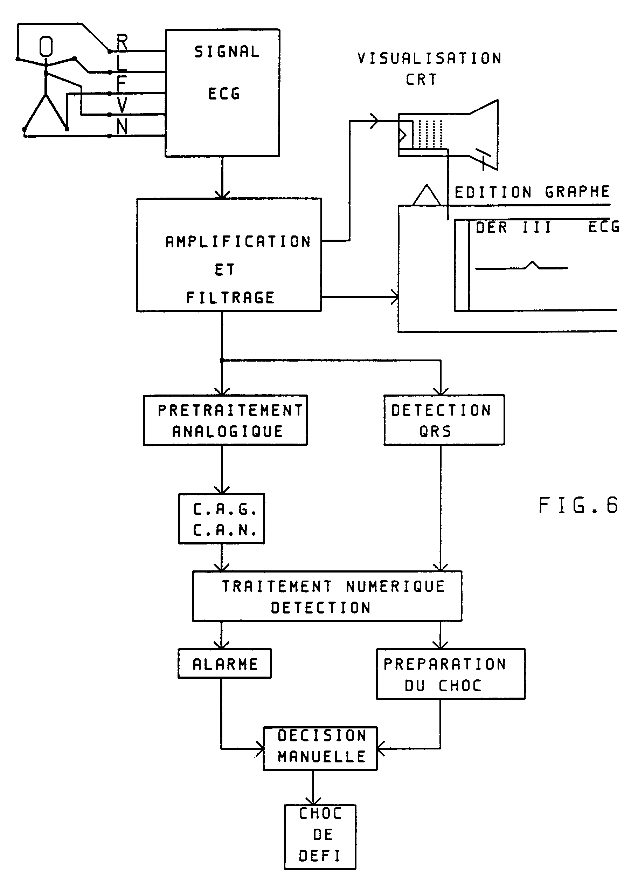

. la figure 5 est un électrocardiogramme de tachycardie ventriculaire ;

. la figure 6 est le schéma synoptique du procédé de détection d'un état pathologique cardiaque à risque selon la variante de base de l'invention ;

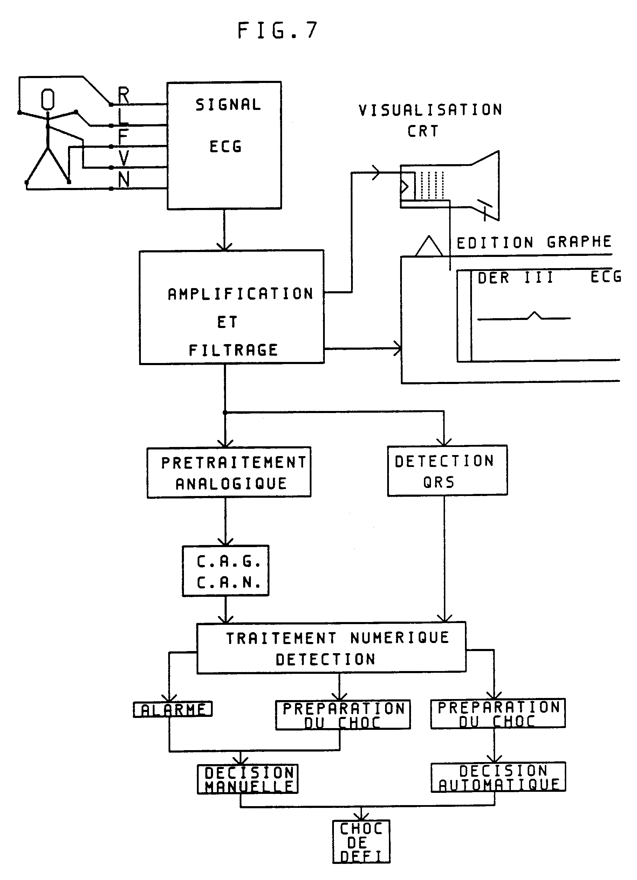

. la figure 7 est le schéma synoptique du procédé de détection d'un état pathologique cardiaque à risque selon la variante de base améliorée par la décision automatique de choc et une analyse complémentaire ;

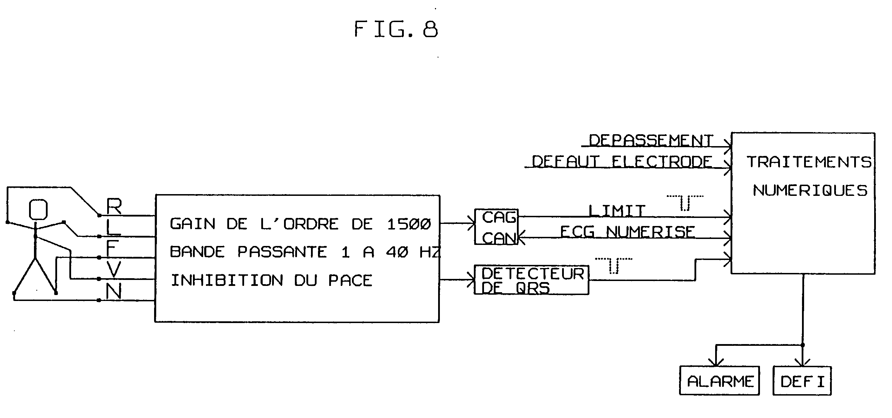

. la figure 8 est le schéma synoptique du procédé de détection d'un état pathologique cardiaque à risque selon une variante sans chaîne d'acquisition ;

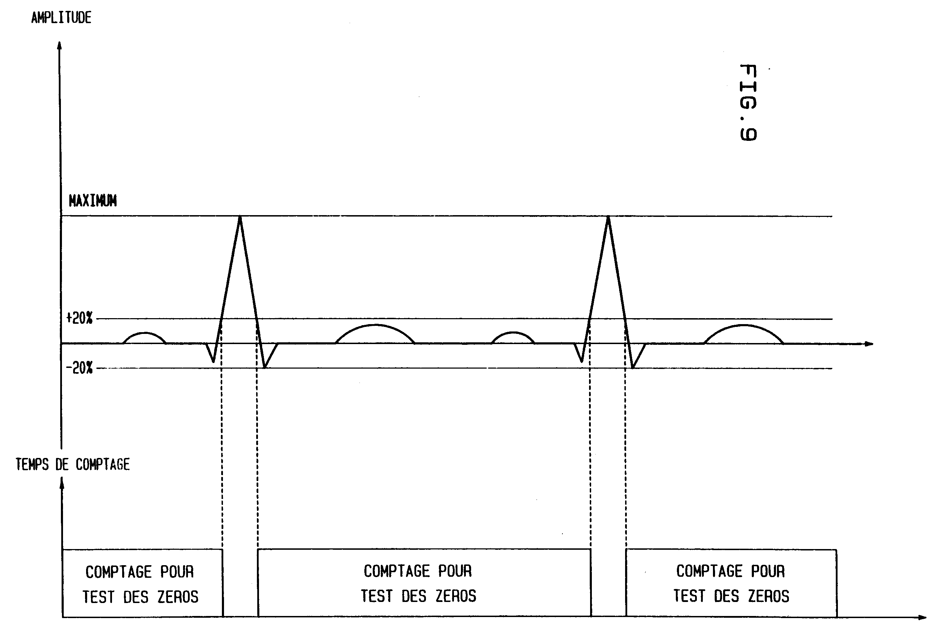

. la figure 9 est un double graphique explicatif du critère d'analyse appelé "critère zéro" ;

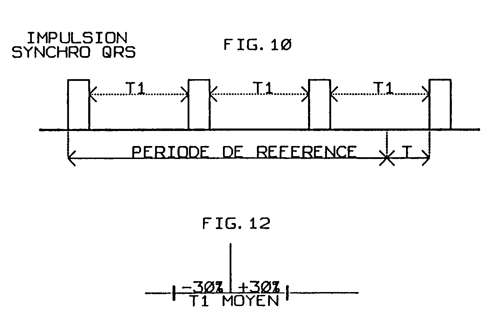

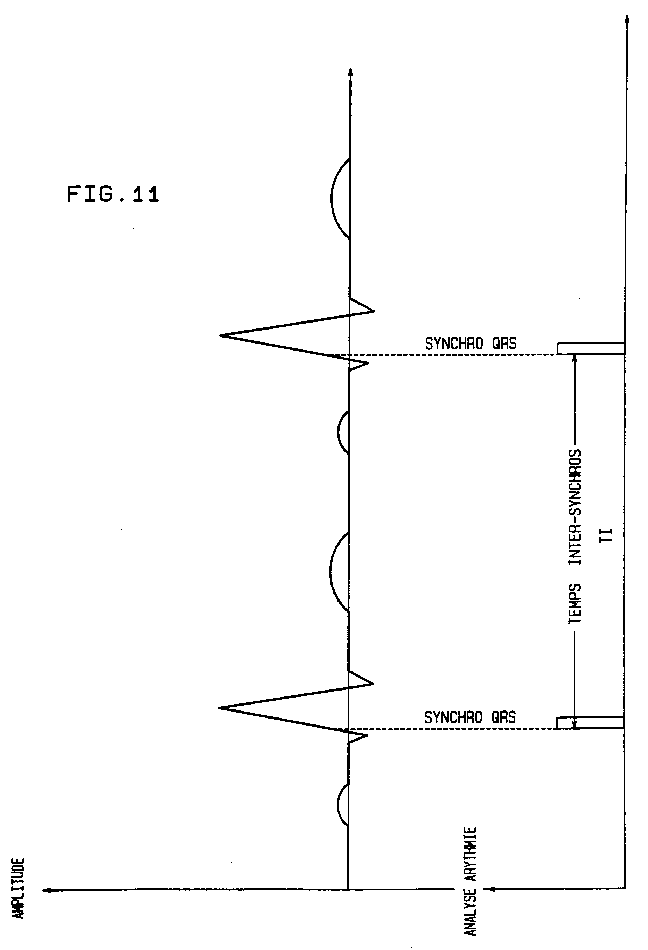

. la figure 10 est un schéma illustrant la définition du critère de fréquence cardiaque FC ;

. la figure 11 est un double graphique montrant la position des impulsions de synchro QRS pour la mise en oeuvre du critère d'analyse appelé "critère d'arythmie" ;

. la figure 12 est un schéma illustrant la définition du critère d'analyse appelé "critère d'arythmie" ;

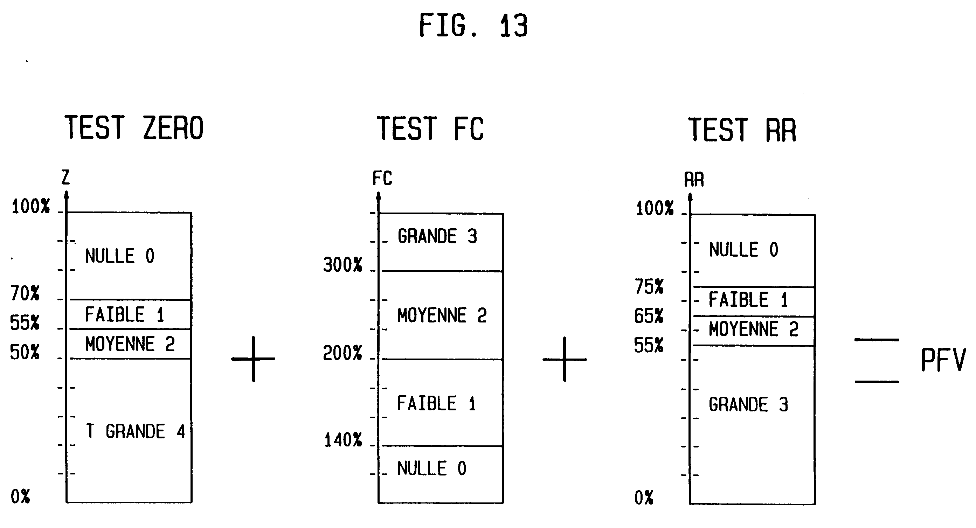

. la figure 13 est un schéma composite montrant les échelles de pondération associées aux trois tests principaux ;

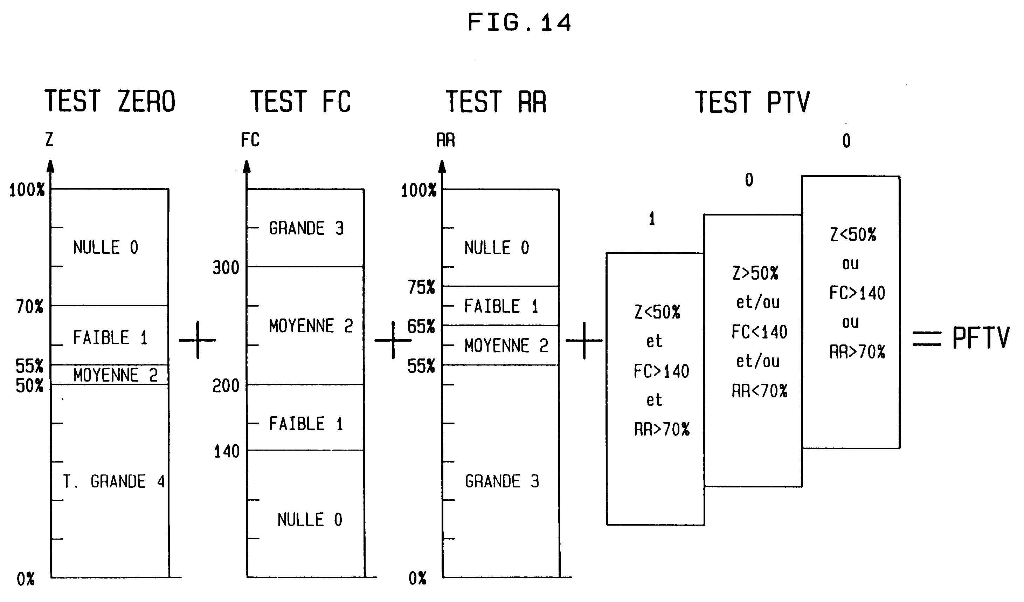

. la figure 14 est un schéma composite montrant les échelles de pondération associées aux quatre tests du procédé amélioré ;

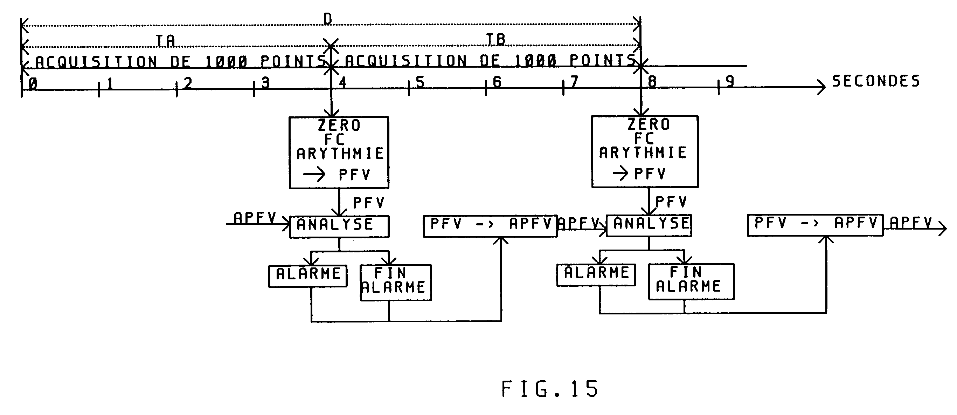

. la figure 15 est le schéma illustrant la chronologie des événements sur une durée d'analyse et de mesure formée de deux périodes élémentaires successives et juxtaposées TA et TB ;

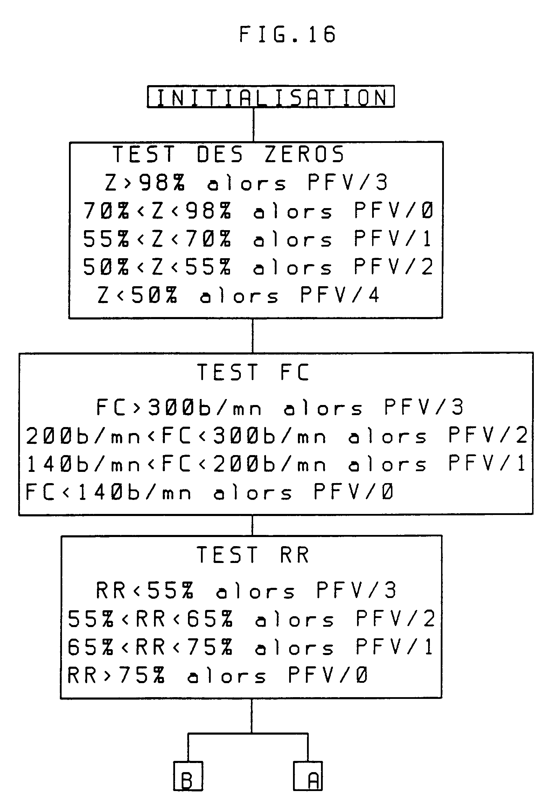

. la figure 16 est l'organigramme de l'algorithme de passage aux trois tests principaux du procédé selon l'invention ;

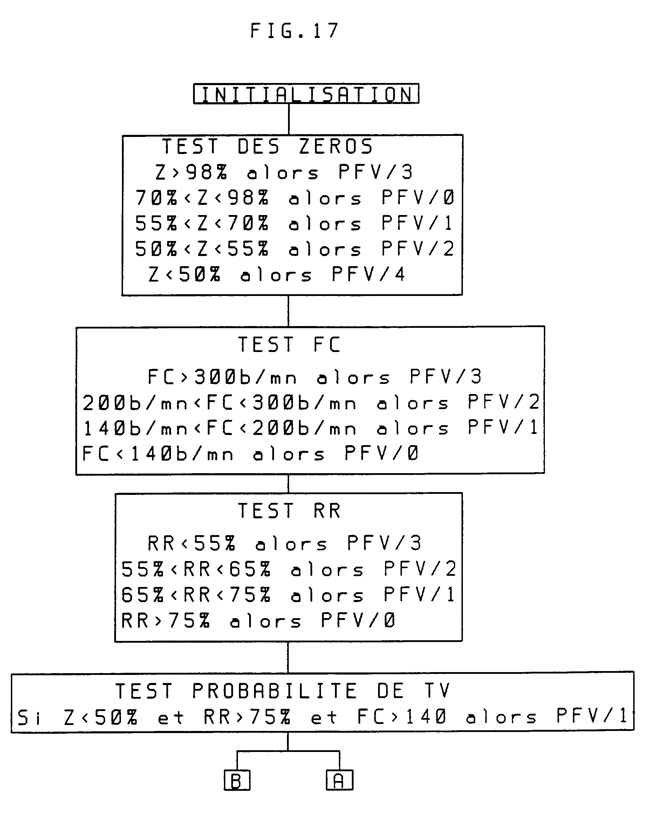

. la figure 17 est l'organigramme de l'algorithme de passage aux quatre tests du procédé amélioré selon l'invention ;

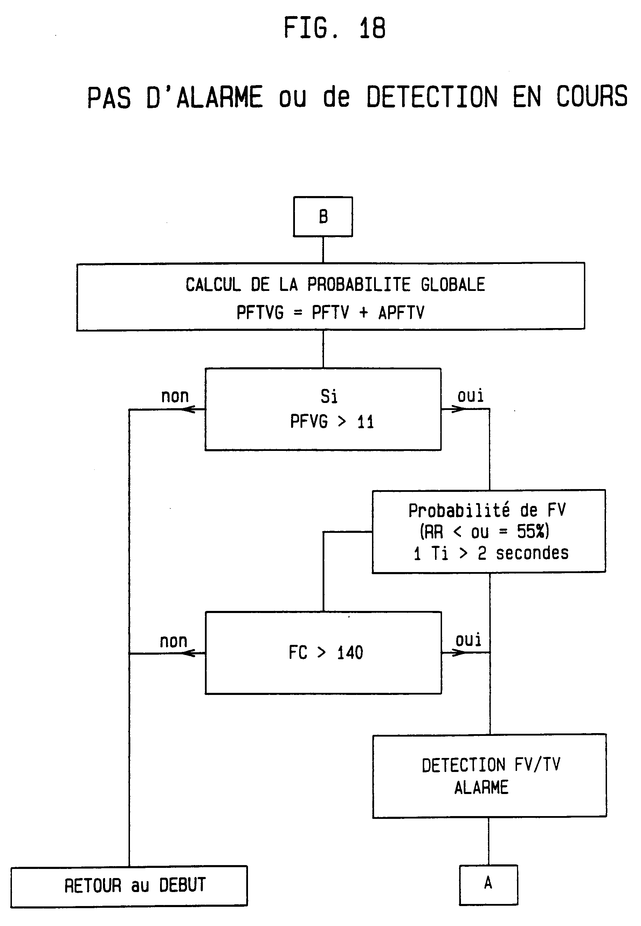

. la figure 18 est l'organigramme de la suite de l'algorithme selon la variante de base améliorée à quatre tests lorsqu'il n'existe pas d'alarme ou de détection en cours ;

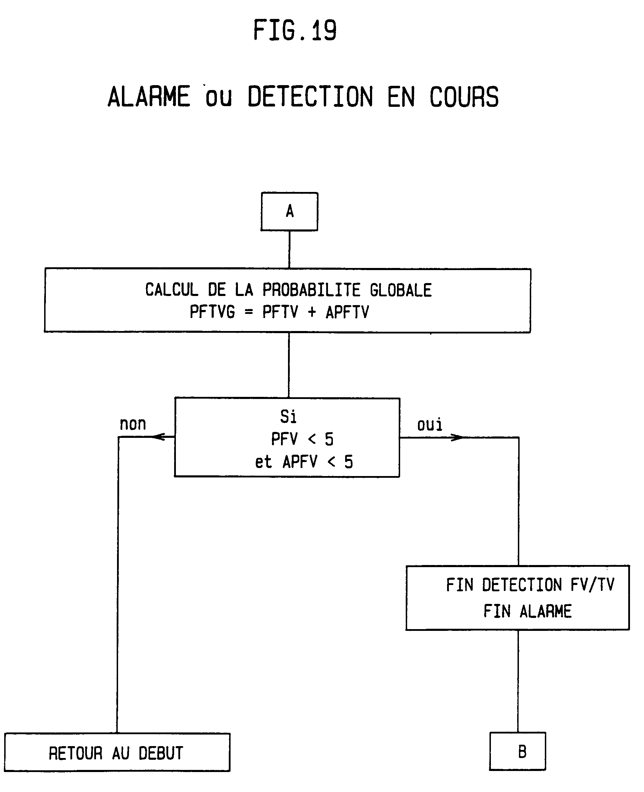

. la figure 19 est l'organigramme de la suite de l'algorithme selon la variante de base améliorée à quatre tests lorsqu'il existe une alarme ou une détection en cours ;

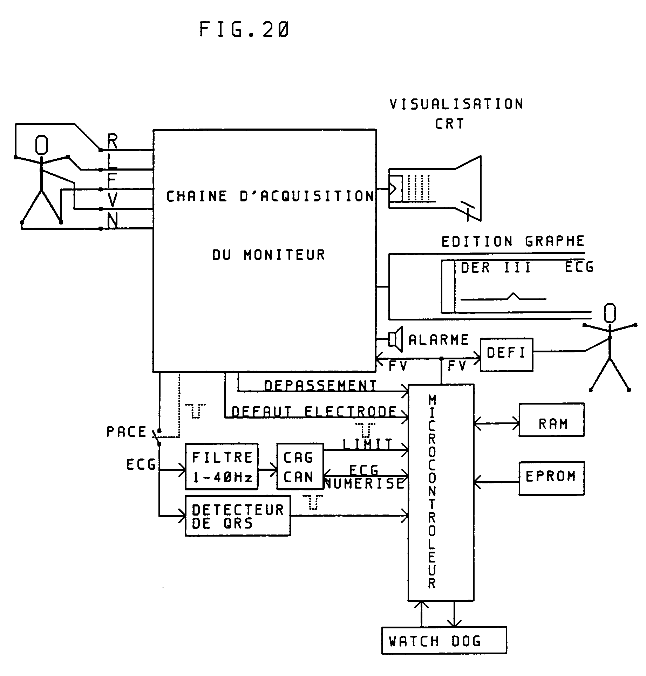

. la figure 20 est un schéma fonctionnel synoptique de l'appareil mettant en oeuvre le procédé selon l'invention ;

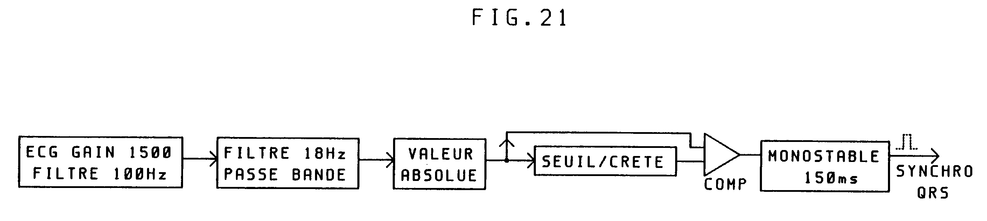

. la figure 21 est le schéma de principe du détecteur des complexes QRS et la formation de l'impulsion de synchro QRS.

A titre préliminaire, on précisera maintenant les diverses notions et définitions utiles pour la clarté et la bonne compréhension de la description effectuée ci-après.

Le signal électrocardiologique se traduisant par un électrocardiogramme, en abrégé ECG, est prélevé sur le corps du patient par des électrodes externes en contact avec la peau. Si les électrodes de défibrillation sont collables, on peut les utiliser pour prélever le signal ECG.

Les signaux électrocardiographiques d'un coeur sain en activité normale sont répétitifs et présentent une forme d'onde répétitive connue constituée par plusieurs segments dont les points caractéristiques sont appelés classiquement P, Q, R, S et T, représentés sur la figure 1.

L'onde P correspond à la dépolarisation des oreillettes. L'intervalle PQ représente le temps de conduction auriculo-ventriculaire. Le complexe QRS correspond à la dépolarisation ventriculaire (propagation dans les ventricules). La repolarisation des ventricules s'effectue de S à T (segment ST) et pendant l'onde T.

Le complexe QRS est un des éléments caractéristiques de l'électrocardiogramme d'activité cardiaque normale. Il sera utilisé dans le procédé décrit ci-dessous.

Impulsion synchro QRS :

Le terme "impulsion Synchro QRS" désigne l'impulsion formée à partir du complexe QRS réel par comparaison avec un seuil de valeur fixe ou variable, par exemple variable dans le cas d'application envisagé ici.

Son rôle consiste à montrer l'existence d'un complexe QRS ou d'une forme voisine très proche.

De plus, il constitue une référence temporelle pour le déclenchement du choc de défibrillation dans le cas de la tachycardie ventriculaire.

Interbattement :

Le terme "interbattement", en abrégé "Ti", est attribué à la durée séparant deux impulsions de synchro QRS.

PACE :

Par convention, le terre PACE signifie l'inhibition de l'impulsion de stimulation.

Comme il apparaît sur les électrocardiogrammes représentés sur les figures 4 et 5, les signaux électrocardiographiques caractéristiques d'une fibrillation et d'une tachycardie ventriculaires se traduisent par une perte totale du caractère reproductible et périodique.

En ce qui concerne la fibrillation, on note en plus l'allure arythmique et aléatoire du signal.

Par contre, les signaux ECG de tachycardie ventriculaire montrent une pseudo-périodicité à fréquence élevée.

Le procédé de surveillance, de détection et de reconnaissance d'un état de fibrillation ventriculaire ou de tachycardie ventriculaire à partir du signal électrocardiologique selon l'invention procède de l'idée générale inventive qui consiste à prétraiter, à numériser les signaux électrocardiographiques après un découpage à une fréquence déterminée, à mesurer à partir de ce signal des valeurs répondant aux trois critères de zéro, de fréquence cardiaque, d'arythmie, à affecter périodiquement à ces valeurs représentatives de ces trois critères des paramètres variables de probabilité, fonction de leurs niveaux relevés sur l'échelle de pondération propre à chaque critère pendant deux périodes élémentaires consécutives d'analyse et à faire correspondre les mesures sur chaque période élémentaire à une probabilité élémentaire par les échelles de pondération, chaque probabilité élémentaire de chaque période étant additionnée à la précédente pour former une probabilité globale fonction de ces critères afin de reconnaître, selon un facteur de probabilité donné, un état pathologique à risques et déclencher une alarme.

De plus, le procédé est complété par un critère supplémentaire spécifique à la tachycardie ventriculaire.

Après une phase d'acquisition connue, réalisée dans les moniteurs classiques, les signaux ECG subissent un prétraitement analogique par un filtrage dans un filtre de bande passante 1-40Hz avec suppression de la composante continue. Ce filtrage a essentiellement pour but de débarrasser ces signaux des parasites et des composantes harmoniques gênant le traitement des signaux. Le filtrage est suivi d'un contrôle automatique de gain CAG de manière à présenter l'ECG toujours à pleine échelle au convertisseur analogique numérique CAN.

Les signaux analogiques sont ensuite échantillonnés puis convertis en signaux numériques dans le convertisseur CAN à une fréquence d'échantillonnage égale à 250 Hz pendant une durée globale d'analyse D, par exemple de 8 secondes, formée de deux périodes élémentaires successives d'échantillonnage et d'analyse TA et TB, par exemple d'une durée de 4 secondes chacune, correspondant par conséquent à 1.000 échantillons chacune.

Le contrôle automatique de gain CAG est une analyse optimale de la valeur maximale, et a pour but d'utiliser toujours la pleine échelle du convertisseur réalisant l'étape de conversion en signaux numériques.

On comprendra par contrôle automatique de gain, abrégé CAG, lié à la conversion analogique/numérique CAN, une opération qui consiste à utiliser l'amplitude maximale du signal ECG pour une valeur de pleine échelle du convertisseur.

Ainsi, quelle que soit l'amplitude du maximum du signal ECG, la même valeur numérique maximale (pleine échelle) sera utilisée afin de disposer d'un signal exploitable dans tous les cas.

Tout se passe comme si le convertisseur s'adaptait à l'amplitude du signal ECG.

Par exemple, pour une amplitude QRS de deux volts, celle-ci sera décomposée en 256 niveaux équidistants et deux volts correspondront à la pleine échelle numérique. Selon le contrôle automatique de gain visé, une amplitude maximale QRS de deux volts aura la même valeur numérique maximale qu'une amplitude maximale d'un volt, les intermédiaires subissant une transposition proportionnelle.

On sait par ailleurs que le signal analogique d'ECG prétraité a une amplitude habituelle se situant selon les sujets entre 0,2 mV et 5 mV. On sait aussi que pour les amplitudes faibles vers 0,2 mV, le signal ECG n'est plus exploitable correctement. Il est nécessaire de prévoir un signal d'inhibition du détecteur, en abrégé LIMIT sur la figure 20, qui valide l'alarme, signalant ainsi la non-possibilité de détection.

Parallélement au prétraitement analogique et à la numérisation, les signaux analogiques issus de l'étape d'acquisition subissent une détection du complexe "QRS" en vue de la formation de l'impulsion de synchro QRS, comme on le verra ci-après.

Formation de l'impulsion de synchro :

L'impulsion de synchro QRS est formée par un détecteur de QRS constitué de la chaîne des modules suivants représentée sur la figure 21 :

| . amplification-filtrage à 100 Hz de coupure et 1.500 de gain | GAIN FILTRE 100 Hz |
| . filtre passe-bande centré sur 18 Hz | PASSE-BANDE 6 Hz |
| . module de valeur absolue | VALEUR ABSOLUE |
| . module seuil/crête | SEUIL/CRETE |
| . module comparateur | COMP |
| . déclencheur d'impulsion | MONOSTABLE |

Le détecteur du complexe QRS est basé sur l'analyse de la réponse d'un filtre passe-bande de 18 Hz de largeur 6 Hz. C'est la valeur absolue du signal qui est analysée. Si cette valeur absolue dépasse un seuil fonction de l'amplitude crête du dernier complexe QRS et d'un seuil fixe, une impulsion de synchro QRS est émise indiquant par sa présence que le complexe QRS est reconnu.

Ainsi, la présence d'impulsions de synchro QRS traduit l'existence de complexes QRS sur le signal ECG analysé.

Les signaux numériques issus de l'étape de prétraitement analogique et numérique et de l'étape de détection QRS sont traités dans une unité de traitement numérique qui consiste en diverses mesures, des phases de calculs et d'analyses numériques par rapport à certains critères prédéterminés et variables selon des séquences spécifiques décrites plus loin, analyses qui conduisent à la reconnaissance d'un état pathologique dangereux selon une probabilité donnée, déterminée par lesdits critères mais modifiable, et au déclenchement voulu ou automatique d'une alarme.

La sélection des critères pouvant être utilisés pour la reconnaissance d'états pathologiques cardiaques dangereux résulte de multiples travaux et essais.

En vue de la reconnaissance d'un état de fibrillation ventriculaire, la méthode et les critères sélectionnés utilisés sont exposés ci-après :

. l'analyse porte sur les échantillons d'une durée D décomposée en deux périodes successives TA et TB juxtaposées de 4 secondes chacune ;

. les trois critères primordiaux sont : un critère "des zéros", un critère de "fréquence cardiaque" FC, un critère d'"arythmie" RR ;

. on ajoute un critère de tachycardie ventriculaire rapide afin d'augmenter la sensibilité et la fiabilité de la détection pour cette pathologie cardiaque ;

. la définition de ces critères fait qu'ils ne sont pas uniquement VRAI ou FAUX mais peuvent avoir des états intermédiaires.

Après une période élémentaire d'analyse TA ou TB, par exemple de 4 secondes, la valeur élaborée de probabilité élémentaire sur cette période d'un cas de fibrillation ventriculaire PFV est incrémentée en additionnant la probabilité élémentaire de la période immédiatement antérieure APFV pour déterminer une probabilité globale liée à la durée D de deux périodes d'analyse juxtaposées TA et TB. Cette probabilité globale : PGV = PFV + APFV est utilisée pour déclencher l'alarme.

Afin d'éviter les séquences anormalement trop courtes ou les artefacts, c'est la somme de cette probabilité de fibrillation ventriculaire PFV et de la probabilité immédiate antérieure de fibrillation ventriculaire APFV calculée sur la période précédente TA qui déterminera le déclenchement de l'alarme.

Afin de bien comprendre ce qui suit, on définira maintenant de façon simple les trois critères utilisés, à savoir : critère des zéros Z, critère de fréquence cardiaque FC, critère d'arythmie RR, et un quatrième critère optionnel, à l'aide des figures de 9 à 14.

1. Le critère des zéros "Z" :

La sélection de ce critère a été guidée par les considérations suivantes.

L'étude statistique d'une activité cardiaque normale a permis de constater que, sur un ECG, 60 % des points sont situés en niveau en-dessous de + ou - 20 % de la valeur maximale de l'ECG normal (voir figure 9). Le comptage de toutes ces valeurs donne une notion de la morphologie du signal.

Ce critère a été encore amélioré de la façon suivante :

. le maximum de l'ECG numérisé correspond toujours à la pleine échelle de conversion grâce au contrôle automatique de gain CAG ;

. un seuil établi à + ou - 20 % du maximum permet de supprimer le bruit pour les signaux de faibles amplitudes et atténue fortement l'effet des parasites et ondes téléphoniques et radioélectriques ;

. une limite inférieure (par exemple de 0,2 mV), en-dessous de laquelle aucun traitement ne peut plus être fait, valide la détection ;

. ce critère n'est pas vrai ou faux mais il prend en compte un certain nombre de valeurs en fonction du nombre de zéros comptés, ces valeurs ayant été définies statistiquement.

Avec plus de 70 % de valeurs proches de zéro, la probabilité d'avoir un ECG anormal est nulle. De 70 à 50 %, la morphologie est différente d'un rythme normal sinusal et un cas d'urgence est probable. Endessous de 50 %, la probabilité est grande.

Le critère des zéros met en oeuvre le comptage du nombre de points d'échantillonnage dont le niveau est proche du zéro. On appelle proche du zéro les points d'échantillonnage dont la valeur est inférieure à + ou - 20 % du maximum de l'ECG, car en cas de fibrillation ventriculaire FV et de tachycardie ventriculaire TV, le nombre de points proches du zéro devient faible.

On définit précisément et mathématiquement le critère des zéros de la façon suivante :

Il s'agit du pourcentage du nombre de points échantillonnés pendant une période élémentaire d'analyse, dont le niveau de tension est situé à + ou - 20 % du niveau maximum de tension de l'ECG par rapport au nombre total de points échantillonnés inférieur à 50 % du nombre total (figure 9).

On définit cette valeur par Z.

Le critère zéro rend compte de la largeur du complexe QRS.

Exemple :

- ECG normal : on trouve une valeur Z supérieure à 60 % ;
- TV et FV correspondent à une valeur Z inférieure à 50 %.

2. Le critère de fréquence cardiaque "FC" :

Il se rapporte principalement aux tachycardies ventriculaires.

Il est défini par la formule suivante :

$$FC = 60 \times (N + 1) / (T + 2)$$

où

N = nombre d'impulsions de synchro QRS comptées pendant une période de référence ;

T = durée entre la fin de la période de référence et la prochaine impulsion ce synchro QRS (figure 10).

Exemple d'application de la formule :

On compte pendant deux secondes (période de référence) trois impulsions de synchro et la prochaine impulsion de synchro arrive après une seconde.

$$N = 3 \text{ et } T = 1$$

En appliquant la formule ci-dessus, on trouve :

FC = 80 battements par minute

Cette fréquence cardiaque est à rapprocher d'une fréquence cardiaque réelle de 80 battements par minute. En principe, cette fréquence calculée est égale à la fréquence réelle.

Bien qu'il soit difficile de définir un seuil au-dessous duquel une alarme doit être émise, l'échelle des probabilités croissante est la suivante :

. de 150 à 200 b/mn la probabilité est faible

. de 200 à 300 b/mn, elle augmente ;

. au-delà de 300 b/mn, elle est maximale.

3. Le critère d'arythmie "RR" :

Le critère d'arythmie se traduit par un degré d'arythmie RR.

Il caractérise le phénomène aléatoire d'une fibrillation ventriculaire, les autres rythmes cardiaques étant assez réguliers.

On définit le degré d'arythmie comme le rapport en pourcentage du nombre d'interbattements sur dix parmi les dix derniers interbattements qui se situent en valeur relative à + ou - 30 % de la valeur de l'interbattement moyen, en abrégé "Ti moyen" (figure 12).

Exemples :

- La fibrillation ventriculaire FV :

Elle se caractérise par un signal irrégulier. Si le signal d'arythmie est faible, par exemple fortement inférieur à 50 %, il engendrera un fort coefficient de probabilité de fibrillation ventriculaire PFV.

- La tachycardie ventriculaire :

Elle se caractérise par un signal régulier. Si le degré d'arythmie est fort, par exemple supérieur à 50 %, il engendrera un faible coefficient de probabilité de fibrillation ventriculaire PFV et inversement une forte probabilité de tachycardie ventriculaire.

Ce critère permet de discriminer une fibrillation ventriculaire en raison de son caractère essentiellement aléatoire par rapport aux autres pathologies du rythme cardiaque, de nature assez régulière.

4. Le quatrième critère "PTV" :

Afin d'augmenter la sensibilité du détecteur pour la tachycardie ventriculaire rapide, on prévoit selon l'invention un quatrième critère.

Selon ce critère, les trois conditions suivantes doivent être remplies simultanément :

. critère des zéros "Z" inférieur à 50 % ;

. fréquence cardiaque supérieure à 140 b/mn ;

. degré d'arythmie RR supérieur à 75 % ;

Ces trois conditions sont remplies systématiquement pour la tachycardie ventriculaire rapide que l'on cherche à détecter.

Ce critère est mis en oeuvre par le quatrième test. Il est totalement transparent pour la fibrillation ventriculaire qui présente d'autres caractéristiques typiques.

Ce test aboutit à augmenter la probabilité d'un certain poids pour la tachycardie ventriculaire rapide.

Afin d'utiliser une autre référence temporelle, l'analyse de l'arythmie est faite à la fin de 4 secondes et sur les 10 derniers intervalles séparant deux signaux de synchro QRS successifs, ces intervalles étant appelés "temps interbattement" ou Ti.

Après avoir calculé la moyenne de ces temps Ti, chaque interbattement est analysé et comparé à la moyenne. Une tolérance de 30 % autour de cette moyenne détermine le nombre des temps Ti qui sont proches de la moyenne, ce nombre correspond au degré ou critère d'arythmie.

Statistiquement, avec 8 sur 10 intervalles interbattements autour de 30 % de la moyenne, le rythme est considéré comme régulier, en-dessous, le rythme est irrégulier et peut correspondre à un cas d'urgence.

Pour ce critère, les fibrillations auriculaires ont un grand coefficient mais ne provoquent pas d'alarmes, ces coefficients ne sont donc pas pris en compte par le détecteur selon l'invention.

Les signaux ECG échantillonnés et numérisés sur une durée totale D de deux périodes consécutives d'échantillonnage et d'analyse sont traités numériquement dans un microprocesseur utilisé en microcalculateur selon les critères choisis déjà indiqués, de zéro Z, de fréquence cardiaque FC, d'arythmie RR et pour la variante améliorée selon un critère spécifique de tachycardie ventriculaire PTV.

Les 1.000 échantillons de chaque période élémentaire successive TA et TB de 4 secondes chacune sont comptabilisés et analysés à la fin de chaque période par rapport aux critères choisis.

Ainsi :

. on détecte une valeur dite "Z" à utiliser pour le test des zéros;

. on élabore une valeur "FC" représentative de la fréquence cardiaque ;

. on calcule une valeur "RR" représentative de l'arythmie ;

. on attribue périodiquement à chaque valeur de mesure sur une période élémentaire une pondération donnée par une échelle de pondération propre à chaque test pour former une probabilité élémentaire ;

. on somme sur deux périodes d'analyse successives TA et TB les probabilités élémentaires en vue de trouver une probabilité globale déterminante liée à une durée d'analyse qui déclenchera ou non l'alarme selon sa position par rapport à un seuil prédéterminé.

La reconnaissance d'un état de fibrillation ventriculaire ou de tachycardie ventriculaire s'effectue en permanence à partir de l'analyse des trois critères ou des quatre critères (variante améliorée) sur la durée totale d'analyse, soit dans l'exemple huit secondes On convient que si la probabilité globale PFG ou PFVG (variante améliorée) de PFV et APFV est supérieure à 11, l'état de fibrillation ventriculaire ou de tachycardie ventriculaire est reconnu par le micro contrôleur et une alarme est émise.

Cependant 10 synchros sont nécessaires pour pouvoir faire une détection d'état physiologique d'urgence.

La fin de la détection est obtenue pour 2 valeurs successives inférieures à 5 et l'alarme s'arrête (figure 19).

Le procédé peut encore se poursuivre par une étape supplémentaire qui consiste en une étape de "proposition de choc" qui se traduit par la mise en charge des condensateurs de défibrillation et la préparation de l'appareil qui délivre le choc électrique de défibrillation afin que l'opérateur puisse intervenir plus rapidement encore sur le patient.

Selon une autre variante du procédé, on peut supprimer l'étape du prétraitement dans la mesure où l'étape d'acquisition de l'ECG délivre déjà un signal de bande passante adaptée (figure 8).

On donnera ci-après un exemple des échelles de pondération des tests utilisées ainsi qu'un descriptif du fonctionnement dans les deux cas de fibrillation et de tachycardie ventriculaires.

Les figures 13 et 14 illustrent les échelles de pondération des tests utilisés dans les deux variantes du procédé, qui sont, comme déjà indiqué, les suivantes : test de zéro (abrégé : test zéro), test de fréquence cardiaque (abrégé : test FC), test d'arythmie (abrégé : test RR) et test complémentaire de probabilité de tachycardie ventriculaire (abrégé : test PTV).

Ces figures ont pour but de montrer clairement les coefficients de pondération affectés aux différentes valeurs selon leur niveau pour déterminer une probabilité de fibrillation ventriculaire PFV, après deux périodes élémentaires successives d'analyse TA et TB, la probabilité de la période d'analyse immédiatement antérieure étant déterminée de la même façon et référencée APFV.

Pour chaque test, un coefficient de pondération est attribué, selon la zone d'amplitude où se trouve le résultat sur l'échelle de pondération de ce test.

- <u>test des zéros</u> :

On attribue à la valeur de probabilité par l'échelle de pondération du test des zéros un coefficient de pondération variable égal par exemple à :

. 4 si le nombre Z pendant la période d'analyse est situé entre 0 % et 50 % ;

. 2 si le nombre Z pendant la période d'analyse est situé entre 50 % et 55 % ;

. 1 si le nombre Z pendant la période d'analyse est situé entre 55 % et 70 % ;

. 0 si le nombre Z pendant la période d'analyse est situé entre 70 % et 100 % ;

EP 0 562 021 B1

. 3 au-delà.
- test de fréquence cardiaque FC :
  On attribue à la valeur de probabilité par l'échelle de pondération du test de fréquence cardiaque FC un coefficient de pondération variable égal par exemple à :
    . 0 si la fréquence cardiaque pendant la période d'analyse est inférieure à 140 b/mn (probabilité nulle) ;
    . 1 si la fréquence cardiaque pendant la période d'analyse est située entre 140 et 200 b/mn (probabilité faible) ;
    . 2 si la fréquence cardiaque pendant la période d'analyse est située entre 200 et 300 b/mn (probabilité moyenne) ;
    . 3 au-delà (probabilité forte).
- test d'arythmie RR :
  On attribue à la valeur représentant la probabilité par l'échelle de pondération du test d'arythmie RR un coefficient de pondération variable égal par exemple à :
    . 3 si le degré d'arythmie pendant la période d'analyse est inférieur ou égal à 50 % ;
    . 2 si le degré d'arythmie pendant la période d'analyse est situé entre 55 % et 65 % ;
    . 1 si le degré d'arythmie pendant la période d'analyse est situé entre 65 % et 75 % ;
    . 0 si le degré d'arythmie pendant la période d'analyse est situé entre 75 % et 100 %.

On pourrait aussi bien indiquer seulement que dans telle ou telle zone de valeurs, la probabilité est nulle, faible, moyenne ou grande, ceci afin de ne pas se limiter par l'indication de chiffres.
- quatrième test "PTV" :
  Pour augmenter la sensibilité et la fiabilité de la détection dans le cas d'une tachycardie ventriculaire, on procède à un test complémentaire dit test de probabilité de tachycardie ventriculaire PTV" (figure 14).

Ce test complémentaire consiste à vérifier systématiquement si les trois conditions suivantes sont satisfaites simultanément :
    . peu de valeurs zéro dénommées Z ;
    . rythme cardiaque régulier déterminé par une valeur faible de RR ;
    . la fréquence cardiaque est supérieure à 140 battements par minute.

Si ces conditions ne sont pas satisfaites simultanément, on augmente la probabilité de zéro (le test est transparent). C'est le cas pour la fibrillation ventriculaire.

Si ces conditions sont satisfaites simultanément, on augmente la probabilité élémentaire d'un poids faible (par exemple 1) pour donner naissance à une nouvelle probabilité globale PFTVG = PFTV + APFTV (figures 14, 18 et 19).

Si nécessaire, on peut déterminer alors une nouvelle limite de la probabilité occasionnant le déclenchement de l'alarme, en l'augmentant par exemple d'une unité.

Pour une meilleure compréhension du procédé et de l'appareil le mettant en oeuvre, on décrire ci-dessus un exemple de détection de fibrillation ventriculaire et un exemple de détection de tachycardie ventriculaire (seuil de probabilité égal à 11).

On suppose l'état pathologique survenant après plusieurs minutes d'activité normale du coeur du patient.


Fibrillation ventriculaire :

Le brutal changement de nature des signaux se traduira immédiatement par des impulsions de synchro QRS irrégulières.

Le test des zéros fera passer la pondération apportée par ce critère d'une valeur 0 à 4 en raison des formes d'onde plus larges.

Le test FC pourra faire passer pendant quelques instants la valeur au dessus de 200 battements par minute ou dans certains cas aucune impulsion ce complexe QRS ne sera générée pendant une durée nécessairement supérieure à 2 secondes.

Le test RR attribuera une valeur de pondération maximale en raison du caractère aléatoire des réponses du détecteur de QRS.

Il en résultera une valeur de probabilité pour une période d'analyse de PFTV + APFTV = 9 par exemple, cette somme devenant égale à 18 lors du cumul avec la valeur de pondération provenant de la période élémentaire suivante d'analyse TA ou TB. Cette probabilité étant supérieure à 11, la fibrillation ventriculaire FV sera reconnue.

Tachycardie ventriculaire rapide :

Le passage des signaux ECG en déformation typique et en rythme accéléré de tachycardie se traduira par un rythme rapide avec des complexes QRS larges.

On a limité volontairement la détection à un rythme supérieur à 140 battements par minute car le danger n'apparait qu'au delà de cette fréquence cardiaque.

Ainsi, si cette fréquence est atteinte, le détecteur réagira de la façon suivante .

Le critère des zéros attribuera une valeur de pondération de 4 due au complexes QRS larges.

Le critère FC apportera une valeur de pondération au moins de 1.

Le critère RR ne contribuera à aucune augmentation de la probabilité.

Pour déclencher l'alarme, c'est le test supplémentaire de probabilité d'une tachycardie ventriculaire PTV qui ajoutera une valeur égale à 1 si toutes les conditions de ce test sont remplies.

Le nombre de la probabilité globale sera égale à 6 pour une première période élémentaire TA d'analyse et égale au double, c'est à dire PFV + APFV = 12 après la deuxième période élémentaire TB.

Conformément au seuil de probabilité établi à 11, ce nombre étant supérieur à 11, l'alarme se déclenchera.

A partir du signal ECG numérisé, on établit des grandeurs Z, FC et RR correspondant aux trois critères, c'est-à-dire conformes aux définitions indiquées ci-dessus.

Grandeur Z :

La grandeur Z destinée au test de zéro est établie par une comparaison, puis un comptage de tous les points échantillonnés dont la valeur de tension correspondant à la valeur de la définition du critère de zéro, à savoir deux dont le niveau de tension est située à + ou - 20 % du niveau maximum de tension de l'ECG par rapport au nombre total de points échantillonnés.

Des mesures comparatives permettent d'isoler le nombre de points défini ci-dessus ; il suffit ensuite de lui donner la valeur relative souhaitée correspondant à sa définition.

Ces opérations sont effectuées dans l'unité de traitement numérique et de calcul.

Cette grandeur est ensuite soumise au test des zéros selon l'échelle pondérale propre à ce critère représentée sur les figures 13 et 14 pour établir une probabilité élémentaire selon l'algorithme général.

Grandeur FC :

Cette grandeur FC destinée au test de fréquence cardiaque est élaborée par comptage du nombre N d'impulsions de synchro QRS pendant une période de référence donnée. On comptabilise également la durée T entre la fin de la période de référence et la prochaine impulsion de synchro QRS.

Le calculateur établit la fréquence cardiaque selon la formule :

$$FC = 60 \times (N + 1) / (T + 2)$$

Cette grandeur est ensuite soumise au test de fréquence cardiaque selon l'échelle pondérale propre à ce critère représentée sur les figures 13 et 14 pour établir une probabilité élémentaire selon l'algorithme général.

Grandeur RR :

Cette grandeur RR représentant le degré d'arythmie répond à la définition indiquée ci-dessus.

On compte à partir de l'impulsion de synchro QRS dix interbattements Ti. On calcule la moyenne de ces dix derniers interbattements. On compte le nombre d'interbattements dont la valeur est située à + ou - 30 % de la moyenne et on calcule le pourcentage de ce nombre d'interbattements par rapport à l'ensemble de ces interbattements Ti de la période de calcul. Par définition, ce nombre est égal à dix.

Cette grandeur est ensuite soumise au test d'arythmie selon l'échelle pondérale propre à ce critère représentée sur les figures 13 et 14 pour établir une probabilité élémentaire selon l'algorithme général.

Algorithme général :

L'algorithme général rend compte de tous les tests envisagés et de l'algorithme final destiné à la prise de décision.

Cet algorithme est représenté sur les figures 16, 17, 18 et 19.

Il est décrit presque entièrement à toutes les périodes élémentaires TA ou TB.

Pour la bonne compréhension, il est nécessaire de se référer également à la figure 15.

Cet algorithme rend compte du déroulement des opérations systématiques du procédé au-delà de l'établissement des grandeurs résultant des mesures comprenant le passage aux différentse échelles de pondération des tests.

L'algorithme débute par une instruction de réinitialisation après chaque période élémentaire d'analyse TA ou TB en vue de le décrire une nouvelle fois.

L'établissement des valeurs Z, FC et RR s'effectue en continu, sur chaque période élémentaire, ces nombres ou grandeurs sont disponibles à la fin de chaque période élémentaire TA et TB.

A la fin de chacune de ces périodes, l'algorithme est décrit en entier.

L'algorithme est décrit une nouvelle fois à chaque fois que les nouvelles valeurs sont disponibles pour établir une nouvelle probabilité globale.

A l'issue de deux périodes élémentaires TA et TB successives, les deux probabilités élémentaires APFV ou APFTV et PFV ou PFTV sont additionnées dans un sommateur en vue d'établir une probabilité globale qui déterminera un état de reconnaissance et proposera une décision de défibrillation.

L'organigramme de l'algorithme a pour but de montrer le détail des tests.

Ceux-ci sont au nombre des quatre principaux suivants : test des zéros, test FC, test RR pour la variante et test de probabilité d'une tachycardie ventriculaire (test PTV) pour le procédé complet.

Ce dernier test est transparent pour une fibrillation ventriculaire, car la valeur ajoutée est nulle.

Par contre, ce test augmente la probabilité globale dans des proportions différentes selon les critères précédents examinés une nouvelle fois individuellement et pris simultanément en compte.

On peut ainsi définir une nouvelle probabilité générale PGTV qui est la probabilité finale après l'examen de tous les critères et le passage à tous les tests sur deux périodes élémentaires successives d'analyse TA et TB. Elle est égale à la somme de deux nouvelles probabilités élémentaires APFTV et PFTV qui sont les probabilités élémentaires après test final PTV :

$$PGTV = APFTV + PFTV$$

On examinera maintenant la suite de l'algorithme général appliqué par le procédé selon l'invention en référence aux figures 18 et 19.

Le premier algorithme (figure 18) a pour but de mettre en oeuvre un test ultime relatif à la tachycardie ventriculaire rapide (FC supérieur à 140 b/mn) que l'on cherche à détecter de la façon la plus sûre possible.

Le second (figure 19) a pour but de surveiller le cas d'alarme et de déterminer si on doit maintenir ou arrêter l'alarme qui s'est déclenchée.

Concernant le premier algorithme, la limite de probabilité choisie, ici le nombre 11, étant dépassée, le cas d'alarme est acquis. Avant de déclencher l'alarme, un test ultime de tachycardie ventriculaire est utilisé. Ce test concerne les deux alternatives suivantes :

1°) la probabilité de fibrillation ventriculaire FV est celle d'un cas où RR est inférieur ou égal à 55 % (faible degré d'arythmie)

2°) un temps d'interbattement sur dix est supérieur à deux secondes

avec un passage ultérieur de contrôle si la fréquence cardiaque est supérieure à 140b/mn.

Ce test ultime est apparu nécessaire pour les cas où l'on est sûr de ne pas être dans une grande probabilité de fibrillation ventriculaire ou dans le cas d'une détection inefficace de QRS (on ne reconnaît plus le complexe QRS dans le signal).

Ainsi, si l'une ou l'autre des conditions alternatives ci-dessus :

. est remplie, on laisse se déclencher l'alarme ;

. n'est pas remplie, on détermine si la fréquence cardiaque est supérieure à 140b/mn :

- si la fréquence cardiaque est inférieure à cette limite, l'alarme n'est pas validéeet on retourne au début de l'algorithme

- si la fréquence cardiaque est supérieure à cette limite, l'alarme est déclenchée sur cette confirmation.

Dans le cas d'une alarme ou d'une détection en cours, la décision de maintenir ou d'arrêter l'alarme résulte de l'algorithme représenté sur la figure 19.

Si sur deux périodes élémentaires successives de mesure et d'analyse TA et TB, la probabilité est inférieure à 5, alors l'alarme donnée est arrêtée et on retourne à l'algorithme de la figure 18.

Dans le cas contraire, on maintient l'alarme et on retourne au début de l'algorithme général.

EP 0 562 021 B1

Il convient de remarquer ici que les mesures et les analyses s'effectuent périodiquement et en permanence. Il s'agit donc d'une surveillance constante. La moindre modification sensible au détecteur selon l'invention de l'état cardiaque du patient modifiera ou non quasi instantanément l'état d'alarme dans lequel il se trouve.

Il est prévu également d'effectuer une analyse complémentaire et confirmative avant le déclenchement automatique de l'alarme.

Après prélèvement du signal ECG sur le patient, on réalise une amplification-filtrage suivie d'un prétraitement analogique, du contrôle automatique de gain CAG et d'une conversion analogique/numérique dans un convertisseur CAN.

Parallèlement au prétraitement analogique et CAG puis CAN, on détecte le complexe QRS et on forme une impulsion de synchro QRS.

Ces informations, signaux et données sont entrés dans un microcalculateur, par exemple à 8 bits, pour divers traitements numériques de calcul et d'analyse par rapport aux tests sur les valeurs Z, FC, RR, test PTV, en vue d'émettre une alarme en cas de reconnaissance de l'état pathologique cardiaque et de la préparation systématique du choc de défibrillation par la charge du condensateur de défibrillation du fibrillateur associé.

Selon la variante de base, la décision du choc appartient au médecin de surveillance ou personnel équivalent (figure 6).

Selon une variante plus complète, la décision du choc est prise automatiquement et donnée dès qu'une analyse complémentaire montre que l'état pathologique se poursuit (figure 7).

On décrira à présent les moyens de mise en oeuvre du procédé à l'aide des figures 20 et 21.

La chaîne d'acquisition reçoit les signaux recueillis par des électrodes placées sur le patient.

La fonction de la chaîne d'acquisition qui peut faire partie d'un moniteur existant est de délivrer à partir d'un signal superposé à des bruits et parasites, un signal d'ECG de 1 à 5 Volts avec une bande passante prédéfinie par les normes soit de 0,05 à 100 Hz. Les moyens utilisés sont classiques et déjà utilisés pour d'autres appareils de surveillance médicale.

Une visualisation par écran et une imprimante pour l'édition graphique font partie de la chaîne d'acquisition.

Le détecteur proprement dit selon l'invention comporte essentiellement des moyens de prétraitement du signal fourni par la chaîne d'acquisition, des moyens de traitement numérique et un dispositif d'alarme.

Les moyens de prétraitement analogique se composent d'un convertisseur analogique - numérique CAN précédé, dans la variante représentée sur la figure 20, d'un filtre dont les valeurs limites de filtrage 1 et 40 Hz environ, ont été justifiées précédemment. Ce filtre a pour but de permettre à l'utilisateur d'adapter la bande passante de sortie de la chaîne d'acquisition aux fréquences d'entrée limitées à 1 et 40 Hz du convertisseur analogique - numérique.

Selon une autre variante, on peut prévoir de remplacer la chaîne d'acquisition standard ci-dessus par une chaîne d'acquisition délivrant un signal dont la bande passante est déjà adaptée, dans ce cas, les moyens de prétraitement ne comporteraient pas de filtre. Eventuellement, on peut prévoir un ou des moyens pour supprimer des signaux issus de détecteurs ou d'appareils autres que le détecteur de fibrillation ventriculaire et de tachycardie ventriculaire selon l'invention, et qui perturberaient le signal d'ECG. A titre d'exemple non limitatif, on a représenté symboliquement avec la mention PACE sur la figure 20 une fonction "interrupteur" destiné à éliminer les impulsions générées par un stimulateur cardiaque.

Les moyens de prétraitement sont composés essentiellement :
.  d'un convertisseur analogique numérique en abrégé CAN
.  d'un contrôleur automatique de gain en abrégé CAG
.  d'un optimiseur de valeur de crête du signal.

L'optimiseur de valeur de crête est un composant électronique classique comportant une diode chargeant un condensateur de valeur crête, si l'amplitude du signal prétraité est plus grande que l'amplitude stockée dans le condensateur. Ce condensateur est choisi avec une constante de temps de charge de 10 millisecondes pour éviter de prendre en compte un parasite de grande amplitude et avec une constante de temps de décharge de quelques secondes (4 ou 8 par exemple) pour permettre de suivre le maximum du signal si l'amplitude de celui-ci diminue.

Le contrôleur automatique de gain CAG permet d'utiliser toujours la pleine échelle du convertisseur analogique numérique CAN. Comme indiqué, on a choisi pour référence de conversion, la valeur absolue de l'amplitude maximale du signal analogique prétraité.

Le moyen de traitement proprement dit est basé sur un micro calculateur, par exemple un microprocesseur type 8 051 INTEL (8 bits) utilisé en microcalculateur qui nécessite peu de composants périphériques : un quartz de pilotage, une EPROM contenant le programme de traitement numérique, une mémoire de

13

travail RAM et un moyen d'autocontrôle dit "watch dog". Le programme de traitement numérique est celui décrit précédemment pour le procédé.

En parallèle avec les moyens de prétraitement, on prévoit un détecteur de complexe QRS spécialement conçu pour délivrer au micro calculateur ou à l'unité de traitement numérique un signal numérique en synchronisme avec chaque complexe QRS détecté sur l'ECG du patient.

On se reportera à présent à la figure 21 pour la description du détecteur de complexe QRS.

Un filtre passe bande de 18 Hz, de largeur de bande 6 Hz élimine les composantes différentes du complexe QRS. Ce complexe pouvant être positif ou négatif suivant la dérivation de l'électrocardiogramme, le moyen redresseur permet de prendre en compte la valeur absolue de la réponse du filtre passe bande 18 Hz pour détecter toujours la première impulsion positive.

La détection du complexe se fait ensuite à l'aide d'un comparateur COMP par dépassement d'un seuil, qui est variable et dépend de l'amplitude maximale du dernier complexe QRS détecté, du temps écoulé depuis ce dernier QRS, et d'une valeur fixe (qui est une valeur minimum de détection). On a trouvé un compromis entre ces trois données qui sont principalement des constantes de temps de charge et de décharge du condensateur. Le signal issu du comparateur est un signal numérique de présence ou d'absence de complexe QRS, il est ensuite traité par un monostable, de période réfractaire de l'ordre de 150 à 200 ms qui empêche le comptage double pour les complexes larges et qui permet de délivrer un signal de synchronisation jusqu'à des fréquences cardiaques supérieures à 300 pulsations par minute.

Selon une variante, le signal d'entrée du détecteur de QRS est prélevé à l'entrée du filtre de prétraitement. Cette disposition n'est pas limitative, le prélèvement pouvant avoir lieu par exemple directement sur la chaîne d'acquisition d'un signal provenant des électrodes de défibrillation collables.

Le détecteur de fibrillation ventriculaire et de tachycardie comporte encore :
- un dispositif d'alarme technique dans le but de valider l'alarme en cas d'anomalie dans la chaîne d'acquisition, prétraitement, traitement, par exemple, lorsqu'une électrode s'est détachée du patient. Ce type d'alarme est symbolisé sur la figure par "défaut électrode".

Le dispositif d'alarme proprement dit consiste en une alarme sonore et/ou un message visuel gérés par exemple par la chaîne d'acquisition mais validée par le signal numérique de sortie du microcalculateur.

Pour la variante de détecteur semi-automatique, le signal numérique de sortie du micro calculateur valide également des moyens de charge dont la fonction est de placer un défibrillateur associé en état de fonctionner dès que l'opérateur lui donnera l'impulsion de commande. Cette variante est représentée en pointillés sur la figure 20.

## Revendications

1. Procédé de surveillance, de détection et de reconnaissance d'un état cardiaque pathologique à risques, à partir des circuits d'acquisition du signal dit ECG, en vue d'une défibrillation, dans lequel après un prétraitement analogique, on échantillonne et on numérise le signal ECG en permanence sur une durée répétitive de mesure et d'analyse, on établit à partir de ce signal numérisé des valeurs de zéro et de degré d'arythmie, on forme parallèlement à partir du signal analogique d'ECG des impulsions de complexe QRS rendant compte de l'existence de complexes QRS et on traite numériquement les valeurs de zéro et de degré d'arythmie pour les durées de mesure et d'analyse pour déterminer la présence ou l'absence de fibrillation, caractérisé en ce qu'il consiste à subdiviser chaque durée D de mesure et d'analyse répétitive en deux périodes élémentaires consécutives identiques TA et TB, à établir des valeurs de critères de zéro Z et de degré d'arythmie RR, ainsi qu'une valeur de critère de fréquence cardiaque FC, pour chaque période élémentaire TA et TB, à affecter à chacune de ces valeurs de critères Z, RR et FC une valeur de probabilité élémentaire propre, fonction des niveaux desdites valeurs et de leur situation sur une échelle de pondération à plusieurs niveaux, par exemple 0, 1, 2; 3 ou 4, propre à chaque critère et, à sommer les probabilités élémentaires précitées de chaque période élémentaire d'analyse immédiate TB avec celles de la période élémentaire d'analyse antérieure TA, pour obtenir, en temps réel et en permanence, une probabilité globale PGV pour chaque durée D de mesure et d'analyse composée de deux périodes élémentaires, permettant, d'une part, de reconnaître un état pathologique nécessitant une défibrillation, par comparaison de la valeur de PGV avec une valeur limite ou seuil de probabilité prédéterminée, et, d'autre part, le cas échéant, de déclencher une alarme d'urgence et de mettre un défibrillateur associé en condition de charge électrique en vue d'une validation de décharge de défibrillation, une valeur de probabilité supplémentaire PTV étant, en outre, additionnée à chaque probabilité élémentaire lorsque la valeur du critère RR est inférieure à une valeur prédéterminée et au moins un temps d'interbattement Ti sur n est supérieure à une durée fixée.

2. Procédé de surveillance selon la revendication 1, caractérisé en ce que la fréquence d'échantillonnage est de 250 Hz et chaque période d'analyse TA et TB de 4 secondes.

3. Procédé de surveillance selon les revendications 1 et 2, caractérisé en ce que l'on forme une impulsion de synchro QRS à partir d'un signal ECG par une amplification-filtrage à 100 Hz de coupure, par un deuxième filtrage passe-bande centré sur 18 Hz, que l'on isole la valeur absolue du signal, que cette valeur absolue est comparée à un seuil variable, que sa situation par rapport au seuil, à la crête et sa valeur absolue sont comparées en vue d'émettre une impulsion de synchro QRS calibrée en amplitude et en durée.

4. Procédé de surveillance selon l'une quelconque des revendications 1 à 3, caractérisé en ce que l'on définit et l'on établit des valeurs Z de zéro représentant le pourcentage du nombre de points échantillonnés pendant une période d'analyse, dont le niveau de tension est situé à + ou - 20 % du niveau maximum de tension de l'ECG par rapport au nombre total de points échantillonnés inférieur à 50 % du nombre total et en ce que l'on établit une valeur de probabilité liée à la valeur Z pondérée par attribution à la valeur de probabilité, à travers une échelle de pondération du test des zéros, d'un coefficient de pondération variable égal par exemple à:
   . 4 si le nombre Z pendant la période d'analyse est situé entre 0 % et 50 %;
   . 2 si le nombre Z pendant la période d'analyse est situé entre 50 % et 55 %;
   . 1 si le nombre Z pendant la période d'analyse est situé entre 55 % et 70 %;
   . 0 si le nombre Z pendant la période d'analyse est situé entre 70 % et 100 %;
   . 3 au-delà.

5. Procédé de surveillance selon l'une quelconque des revendications 1 à 3, caractérisé en ce que l'on définit et l'on établit des valeurs FC de fréquence cardiaque par la formule suivante:

$$FC = 60 \times (N + 1) / (T + 2)$$

où N = nombre d'impulsions de synchro QRS comptées pendant une période de référence;
   T = durée entre la fin de la période de référence et la prochaine impulsion de synchro QRS,
et en ce que l'on établit une valeur de probabilité élémentaire liée à la valeur FC qui est pondérée par attribution à la valeur de probabilité par l'échelle de pondération du test de fréquence cardiaque FC un coefficient de pondération variable égal par exemple à:
   . 0 si la fréquence cardiaque pendant la période d'analyse est inférieure à 140 (probabilité nulle);
   . 1 si la fréquence cardiaque pendant la période d'analyse est située entre 140 et 200 (probabilité faible);
   . 2 si la fréquence cardiaque pendant la période d'analyse est située entre 200 et 300 (probabilité moyenne);
   . 3 au-delà (probabilité forte).

6. Procédé de surveillance selon l'une quelconque des revendications 1 à 3 caractérisé en ce que l'on définit et l'on établit des valeurs RR de degré d'arythmie comme étant le rapport en pourcentage du nombre d'interbattements sur dix parmi les dix derniers interbattements qui se situent en valeur relative à + ou - 30 % de la valeur de l'interbattement moyen "Ti moyen" et en ce que l'on établit une valeur de probabilité élémentaire liée à la valeur RR qui est pondérée par attribution à la valeur représentant la probabilité à travers une échelle de pondération du test d'arythmie RR d'un coefficient de pondération variable égal par exemple à:
   . 3 si le degré d'arythmie pendant la période d'analyse est inférieur ou égal à 50 %;
   . 2 si le degré d'arythmie pendant la période d'analyse est situé entre 55 % et 65 %;
   . 1 si le degré d'arythmie pendant la période d'analyse est situé entre 65 % et 75 %;
   . 0 si le degré d'arythmie pendant la période d'analyse est situé entre 75 % et 100 %.

7. Procédé de surveillance selon l'une quelconque des revendications 1 à 6, caractérisé en ce que l'on ajoute une probabilité supplémentaire, correspondant au critère de tachycardie ventriculaire PTV, si les conditions suivantes sont remplies simultanément:
   . la possibilité de fibrillation ventriculaire est celle du cas où le degré d'arythmie RR est inférieur ou égal à 55 %;
   . un temps d'interbattement Ti sur dix est supérieur à 2 secondes.

15

**8.** Procédé de surveillance selon l'une quelconque des revendications 1 à 7, caractérisé en ce que dans le cas d'un déclenchement d'alarme on procède à un test final qui consiste à vérifier si le degré d'arythmie est inférieur ou égal à 55 % et si le temps d'interbattement est supérieur à 2 secondes au moins pour décider de valider l'alarme en cas de réponse positive et pour décider de valider l'alarme également dans le cas d'une réponse négative si la fréquence cardiaque est supérieure à 140 b/mn.

**9.** Procédé de surveillance selon l'une quelconque des revendications 1 à 8, caractérisé en ce que l'on arrête l'alarme si les probabilités élémentaires sur deux périodes élémentaires successives TA et TB sont toutes les deux inférieures à 5.

**10.** Procédé de surveillance selon l'une quelconque des revendications 1 à 9, caractérisé en ce que l'on prédispose automatiquement au choc le défibrillateur associé dès que les conditions de la décision du choc sont remplies.

## Claims

**1.** Process for monitoring, detecting and recognizing a hazardous pathological cardiac state from circuits for the acquisition of the so-called ECG signal with a view to defibrillation in which, after analog pro-processing, samples are taken and the ECG signal is continuously digitized for a repetitive measurement and analysis duration, values of zero and of the degree of asynchronism are established from this digitized signal, QRS complex pulses accounting for the existence of QRS complexes are concurrently formed from the analog ECG signal and the values of zero and of the degree of asynchronism are processed digitally for the measurement and analysis durations to determine the presence or absence of fibrillation, characterized in that it involves subdividing each repetitive measurement and analysis duration D into two identical consecutive elementary periods TA and TB, establishing values of criteria of zero Z and degree of asynchronism RR as well as a value for cardiac frequency criterion FC, for each elementary period TA and TB, allocating to each of these values of criteria Z, RR and FC a particular elementary probability value depending on the levels of said values and their situation on a weighting scale having several levels, for example 0, 1, 2; 3 or 4, particular to each criterion and adding the aforementioned elementary probabilities of each immediate elementary period of analysis TB to those of the previous elementary period of analysis TA to obtain, in real time and continuously, a global probability PGV for each measurement and analysis duration D composed of two elementary periods allowing, on the one hand, recognition of a pathological state necessitating defibrillation by comparison of the value of PGV with a predetermined limit value or probability threshold and, on the other hand, if applicable, the triggering of an emergency alarm and the placing of an associated defibrillator in an electric charge state with a view to defibrillation discharge validation, an additional probability value PTV also being added to each elementary probability when the value of the criterion RR is lower than a predetermined value and at least one interval time Ti in n is greater than a fixed duration.

**2.** Monitoring process according to Claim 1, characterized in that the sampling frequency is 250 Hz and each analysis period TA and TB is 4 seconds.

**3.** Monitoring process according to Claims 1 and 2, characterized in that a synchronizing pulse QRS is formed from an ECG signal by cut-out amplification/filtering at 100 Hz, by second band pass filtering centred on 18 Hz, in that the absolute value of the signal is isolated, that this absolute value is compared with a variable threshold, that its situation relative to the threshold at the peak and its absolute value are compared in order to deliver a synchronizing pulse QRS calibrated in amplitude and duration.

**4.** Monitoring process according to any one of Claims 1 to 3, characterized in that values Z of zero representing the percentage of the number of points sampled during an analysis period are defined and established, the voltage level of which is located at + or - 20% of the maximum voltage level of the ECG relative to the total number of sampled points lower than 50% of the total number and in that a probability value liked to the value Z weighted by allocation to the probability value is established through a weighting scale of the zero test of a variable weighting coefficient equal, for example, to:
4 if the number Z is between 0% and 50% during the analysis period;
2 if the number Z is between 50% and 55% during the analysis period;
1 if the number Z is between 55% and 70% during the analysis period;

0 if the number Z is between 70% and 100% during the analysis period;
3 beyond.

5. Monitoring process according to any one of Claims 1 to 3, characterized in that cardiac frequency values FC are defined and established by the following formula;

$$FC = 60 \times (N + 1) / (T + 2)$$

wherein N = number of synchronizing pulses QRS counted during a reference period;
    T = duration between the end of the reference period and the next synchronizing pulse QRS,
and in that an elementary probability value is established which is linked to the value FC which is weighted by allocation to the probability value by the weighting scale of the cardiac frequency test FC, a variable weighting coefficient equal, for example, to:
0 if the cardiac frequency is less than 140 during the analysis period (zero probability);
1 if the cardiac frequency is between 140 and 200 during the analysis period (weak probability);
2 if the cardiac frequency is between 200 and 300 during the analysis period (average probability);
3 beyond (high probability).

6. Monitoring process according to any one of Claims 1 to 3, characterized in that values RR of degree of asynchronism are defined and established as being the percentage ratio of the number of intervals in ten from among the ten last intervals which, in relative value are + or - 30% of the value of the average interval "average Ti" and in that an elementary probability value linked to the value RR is established, which is weighted by allocation to the value representing the probability by means of a weighting scale of the test of asynchronism RR of a variable weighting coefficient equal, for example, to:
3 if the degree of asynchronism is less than or equal to 50% during the analysis period;
2 if the degree of asynchronism is between 55% and 65% during the analysis period;
1 if the degree of asynchronism is between 65% and 75% during the analysis period;
0 if the degree of asynchronism is between 75% and 100% during the analysis period.

7. Monitoring process according to any one of Claims 1 to 6, characterized in that an additional probability is added, corresponding to the criterion of ventricular tachycardia PTV if the following conditions are met simultaneously:
the possibility of ventricular fibrillation is that where the degree of asynchronism RR is less than or equal to 55%;
one interval time Ti in ten is greater than 2 seconds.

8. Monitoring process according to any one of Claims 1 to 7, characterized in that, when an alarm is triggered, a final test is carried out which involves checking whether the degree of asynchronism is less than or equal to 55% and whether the interval time is greater than 2 seconds at least to decide to validate the alarm in the event of a positive response and to decide to validate the alarm also in the case of a negative response if the cardiac frequency is higher than 140 b/mn.

9. Monitoring process according to any one of Claims 1 to 8, characterized in that the alarm is stopped if the elementary probabilities in two successive elementary periods TA and TB are both less than 5.

10. Monitoring process according to any one of Claims 1 to 9, characterized in that the associated defibrillator is automatically predisposed for the shock as soon as the conditions of the shock decision are fulfilled.

**Patentansprüche**

1. Verfahren zum Überwachen, Aufspüren und zur Erkennung eines pathologischen Gefährdungszustandes des Herzens, ausgehend von der Erfassung des EKG-Signals, für eine Defibrillation, bei dem man nach einer analogen Vorbehandlung das EKG-Signal während einer sich wiederholenden Meß- und Analysendauer bemustert und digitalisiert, ausgehend von diesem digitalisierten Signal Null-Werte und Werte für den Grad der Arrhythmie festlegt, parallel dazu ausgehend von dem analogen EKG-Signal Impulse des QRS-Komplexes bildet, welche die Existenz von QRS-Komplexen anzeigen und bei dem man die Null-Werte sowie die Werte für den Grad der Arrhythmie für die Meß- und Analysendauern numerisch verarbeitet, um das Vorhandensein oder dag Fehlen einer Fibrillation zu bestimmen, dadurch gekennzeichnet, daß das Verfahren darin besteht, jede sich wiederholende Meß- und Analysendauer in zwei aufeinanderfolgende und identische Elementarperioden TA und TB zu unterteilen, für jede Elementarperiode TA und TB Kennwerte für den Null-Zustand Z und für den Grad der Arrhythmie RR sowie einen Kennwert für die Herzfrequenz FC festzulegen, jedem dieser Kennwerte Z, RR und FC einen eigenen elementaren Wahrscheinlichkeitswert zuzuordnen als Funktion der Niveaus der genannten Werte und ihrer Lage auf einer für jeden Kennwert vorgesehenen Gewichtungsskala mit mehreren Niveaus, zum Beispiel 0, 1, 2, 3 oder 4, und die genannten elementaren Wahrscheinlichkeiten jeder elementaren Analysenperiode TB mit denjenigen der vorangegangenen elementaren Analysenperiode TA aufzusummieren, um In Echtzeit und fortwährend für jede aus zwei Elementarperioden zusammengesetze Meß- und Analysendauer D eine Gesamtwahrscheinlichkeit PGV zu erhalten, welche es einerseits erlaubt, durch Vergleich des Wertes PGV mit einem vorbestimmten Wahrscheinlichkeits-Grenz- oder Schwellenwert einen pathologischen und eine Defibrillation erfordernden Zustand zu erkennen und welche es andererseits erlaubt, gegebenenfalls einen Notfallalarm auszulösen und einen verbundenen Defibrillator in einen elektrisch aufgeladenen Zustand für eine Bestätigung der Defibrillationsentladung zu versetzen, wobei außerdem ein zusätzlicher Wahrscheinlichkeitswert PTV zu jeder elementaren Wahrscheinlichkeit addiert wird, wenn der Kennwert RR unterhalb eines vorgegebenen Wertes und wenigstens ein Impulszwischenzeitraum Ti von n oberhalb einer festgelegten Dauer liegt.

2. Verfahren zum überwachen nach Anspruch 1, dadurch gekennzeichnet, daß die Frequenz der Bemusterung 250 Hz beträgt und jede Analysenperiode TA und TB vier Sekunden dauert.

3. Verfahren zum Überwachen nach den Ansprüchen 1 und 2, dadurch gekennzeichnet, daß man einen QRS-Synchronimpuls ausgehend von einem EKG-Signal erzeugt, durch eine Verstärkung und Filterung mit einer Abschaltung bei 100 Hz, durch einen zweiten, auf 18 Hz zentrierten Bandpassfilter, daß man den Absolutwert des Signals isoliert, daß dieser Absolutwert mit einem variablen Schwellenwert verglichen wird, daß seine Lage bezüglich des Schwellenwertes, bezüglich der Spitze und sein Absolutwert zum Aussenden eines nach Amplitude und Dauer kalibrierten QRS-Synchronimpulses verglichen werden.

4. Verfahren zum überwachen noch einem der Ansprüche 1 bis 3, dadurch gekennzeichnet, daß man Null-Werte Z, welche den prozentualen Anteil widerspiegeln, der Anzahl der Bemusterungspunkte während einer Analysenperiode, deren Spannungsniveau bei plus oder minus 20 % des maximalen Spannungsniveaus des EKG liegt, bezogen auf die Gesamtanzahl der Bemusterungspunkte, auf unter 50 % der Gesamtanzahl definiert und festlegt und daß man einen mit dem Wert Z verknüpften Wahrscheinlichkeitswert festlegt, welcher gewichtet ist durch Zuordnung eines variablen Gewichtungskoeffizienten zu dem Wahrscheinlichkeitswert gemäß einer Test-Gewichtungsskala der Null-Werte, wobei der variable Gewichtungskoeffizient beispielsweise gleich ist:
4, wenn Z während der Analysenperiode zwischen 0 % und 50 % liegt,
2, wenn Z während der Analysenperlode zwischen 50 % und 55 % liegt,
1, wenn Z während der Analysenperiode zwischen 55 % und 70 % liegt,
0, wenn Z während der Analysenperiode zwischen 70 % und 100 % liegt,
ansonsten 3.

5. Verfahren zum Überwachen nach einem der Ansprüche 1 bis 3, dadurch gekennzeichnet, daß man Werte der Herzfrequenz FC durch folgende Formel definiert und festlegt:

$$FC = 60 \times (N + 1) / (T + 2),$$

wobei N = Anzahl der QRS-Synchronimpulse, welche während einer Referenzperlode gezählt werden;
T = Dauer zwischen dem Ende der Referenzperlode und dem nächsten QRS-Synchronimpuls,
und daß man einen mit dem Wert FC verknüpften elementaren Wahrscheinlichkeitswert festlegt, welcher gewichtet wird durch Zuordnung eines variablen Gewichtungskoeffizienten zu dem Wahrscheinlichkeitswert gemäß der Test-Gewichtungsskala für die Herzfrequenz FC, wobei der Gewichtungskoeffizient beispielsweise gleich ist:
0, wenn die Herzfrequenz wahrend der Analysenperlode unter 140 liegt (Wahrscheinlichkeit 0);
1; wenn die Herzfrequenz während der Analysenperiode zwischen 140 und 200 liegt (geringe Wahrscheinlichkeit);

2, wenn die Herzfrequenz während der Analysenperiode zwischen 200 und 300 liegt (mittlere Wahrscheinlichkeit);

ansonsten 3 (hohe Wahrscheinlichkeit)

6. Verfahren zum Überwachen noch einem der Ansprüche 1 bis 3, dadurch gekennzeichnet, daß man Werte für den Grad der Arrhythmie RR definiert und festlegt als das prozentuale Verhältnis der Anzahl der Impulszwischenzeiträume bezogen auf 10 unter den letzten 10 Impulszwischenzeiträumen, die in ihrem relativen Wert bei plus oder minus 30 % des mittleren Wertes der Impulszwischenzeiträume "Ti mittel" liegen, und daß man einen mit dem Wert RR verknüpften elementaren Wahrscheinlichkeitswert festlegt, welcher gewichtet wird, durch Zuordnung eines variablen Gewichtungskoeffizienten zu dem Wert, welcher die Wahrscheinlichkeit gemäß einer Test-Gewichtungsskala für die Arryhthmie RR repräsentiert, wobei der Gewichtungskoeffizient gleich ist:

3, wenn der Grad dar Arrhythmie während der Analysenperiode kleiner oder gleich 50 % ist;

2, wenn der Grad der Arrhythmie während der Analysenperiode zwischen 55 % und 65 % liegt;

1, wenn der Grad der Arrhythmie während der Analysenperiode zwischen 65 % und 75 % liegt;

0, wenn der Grad der Arrhythmie während der Analysenperiode zwischen 75 % und 100 % liegt.

7. Verfahren zum Überwachen nach einem der Ansprüche 1 bis 6, dadurch gekennzeichnet, daß man eine zusätzliche Wahrscheinlichkeit hinzufügt, welche dem Kriterium der ventrikulären Tachykardie PTV entspricht, wenn folgende Bedingungen gleichzeitig erfüllt sind:

Die Möglichkeit der ventrikulären Fibrillation ist diejenige des Falles, in dem der Grad der Arrhythmie RR kleiner oder gleich 55 % ist;

von 10 Impulszwischenzeiträumen übersteigt ein Impulszwischenzeitraum Ti 2 Sekunden.

8. Verfahren zum Überwachen nach einem der Ansprüche 1 bis 7, dadurch gekennzeichnet, daß im Falle der Auslösung eines Alarms ein abschließender Test durchgeführt wird, welcher darin besteht, zu überprüfen, ob der Grad der Arrhythmie kleiner oder gleich 55 % ist und ob der Impulszwischenzeitraum länger als 2 Sekunden ist, um im Falle einer positiven Antwort wenigstens die Bestätigung des Alarms zu beschließen und im Falle einer negativen Antwort, wenn die Herzfrequenz über 140 Schlägen je Minute liegt, gleichermaßen die Bestätigung des Alarms zu beschließen.

9. Verfahren zum überwachen nach einem der Ansprüche 1 bis 8, dadurch gekennzeichnet, daß man den Alarm beendet, wenn die elementaren Wahrscheinlichkeiten während zweier aufeinanderfolgender Elementarperioden TA und TB beide unter 5 liegen.

10. Verfahren zum Überwachen nach einem der Ansprüche 1 bis 9, dadurch gekennzeichnet, daß man den verbundenen Defibrillator automatisch für den Defibrillationsschock vorbereitet, sobald die Bedingungen für die Entscheidung über einen Defibrillationsschock erfüllt sind.

**FIG. 1**

**FIG. 2**

EP 0 562 021 B1

**FIG.3**

**FIG 4**

**FIG. 5**

FIG.6

# FIG.7

FIG.8

R
L
F
V
N

GAIN DE L'ORDRE DE 1500
BANDE PASSANTE 1 A 40 HZ
INHIBITION DU PACE

CAG
CAN

DETECTEUR
DE QRS

DEPASSEMENT
DEFAUT ELECTRODE

LIMIT
ECG NUMERISE

TRAITEMENTS
NUMERIQUES

ALARME    DEF I

EP 0 562 021 B1

FIG.9

AMPLITUDE

MAXIMUM

+20%

-20%

TEMPS DE COMPTAGE

COMPTAGE POUR
TEST DES ZEROS

COMPTAGE POUR
TEST DES ZEROS

COMPTAGE POUR
TEST DES ZEROS

EP 0 562 021 B1

IMPULSION
SYNCHRO QRS

FIG. 10

T1     T1     T1

PERIODE DE REFERENCE     T

FIG. 12

-30%   +30%
T1 MOYEN

FIG.11

# FIG. 13

TEST ZERO

```
Z
100% ─
│
│   NULLE 0
│
70% ─
55% ─  FAIBLE 1
50% ─  MOYENNE 2
│
│   T GRANDE 4
│
0% ─
```

+

TEST FC

```
FC
│   GRANDE 3
│
300% ─
│   MOYENNE 2
│
200% ─
│   FAIBLE 1
│
140% ─
│   NULLE 0
```

+

TEST RR

```
RR
100% ─
│   NULLE 0
│
75% ─  FAIBLE 1
65% ─  MOYENNE 2
55% ─
│
│   GRANDE 3
│
0% ─
```

= PFV

EP 0 562 021 B1

FIG.14

FIG.15

EP 0 562 021 B1

FIG.16

INITIALISATION

TEST DES ZEROS
Z>98% alors PFV/3
70%<Z<98% alors PFV/0
55%<Z<70% alors PFV/1
50%<Z<55% alors PFV/2
Z<50% alors PFV/4

TEST FC
FC>300b/mn alors PFV/3
200b/mn<FC<300b/mn alors PFV/2
140b/mn<FC<200b/mn alors PFV/1
FC<140b/mn alors PFV/0

TEST RR
RR<55% alors PFV/3
55%<RR<65% alors PFV/2
65%<RR<75% alors PFV/1
RR>75% alors PFV/0

B     A

FIG.17

INITIALISATION

TEST DES ZEROS
Z>98% alors PFV/3
70%<Z<98% alors PFV/0
55%<Z<70% alors PFV/1
50%<Z<55% alors PFV/2
Z<50% alors PFV/4

TEST FC
FC>300b/mn alors PFV/3
200b/mn<FC<300b/mn alors PFV/2
140b/mn<FC<200b/mn alors PFV/1
FC<140b/mn alors PFV/0

TEST RR
RR<55% alors PFV/3
55%<RR<65% alors PFV/2
65%<RR<75% alors PFV/1
RR>75% alors PFV/0

TEST PROBABILITE DE TV
Si Z<50% et RR>75% et FC>140 alors PFV/1

B    A

# FIG. 18

## PAS D'ALARME ou de DETECTION EN COURS

```
                    ┌─────┐
                    │  B  │
                    └──┬──┘
   ┌───────────────────┴───────────────────┐
   │     CALCUL DE LA PROBABILITE GLOBALE   │
   │        PFTVG = PFTV + APFTV            │
   └───────────────────┬───────────────────┘
                        │
        non    ┌────────┴────────┐    oui
     ◄─────────┤       Si        ├─────────►
              │    PFVG > 11     │
              └─────────────────┘
                                   ┌────────────────────────┐
                                   │   Probabilité de FV     │
                                   │   (RR < ou = 55%)       │
                                   │   1 Ti > 2 secondes     │
                                   └────────────────────────┘
        non    ┌─────────────────┐    oui
     ◄─────────┤    FC > 140     ├─────────►
              └─────────────────┘
                                   ┌────────────────────────┐
                                   │    DETECTION FV/TV      │
                                   │       ALARME            │
                                   └────────────────────────┘
   ┌──────────────────────┐              ┌─────┐
   │   RETOUR au DEBUT     │              │  A  │
   └──────────────────────┘              └─────┘
```

# FIG.19

## ALARME ou DETECTION EN COURS

# FIG.20

FIG.21

ECG GAIN 1500
FILTRE 100Hz → FILTRE 18Hz
PASSE BANDE → VALEUR
ABSOLUE → SEUIL/CRETE → COMP → MONOSTABLE
150ms → SYNCHRO QRS

EP 0 562 021 B1